# EUROPÄISCHE PATENTANMELDUNG

(11) **EP 3 797 727 A1**
(43) Veröffentlichungstag der Anmeldung: **31.03.2021**
(21) Anmeldenummer: 19200367.1
(22) Anmeldetag: 30.09.2019
(51) Int. Cl.: A61B 42/50

(54) **SYSTEM UND VERFAHREN ZUM EINFÜHREN EINER HAND IN GEÖFFNETEN EINWEGHANDSCHUH UND ANZIEHEN DES EINWEGHANDSCHUHS AN DIE HAND**

(71) Anmelder: Glomatec GmbH, 6005 Luzern (CH)
(72) Erfinder: AREGGER, Raphael, 6005 Luzern (CH); MARCOS, Pedro, 6005 Luzern (CH)
(74) Vertreter: Koelliker, Robert

(57) **Zusammenfassung**

Die vorliegende Erfindung bezieht sich auf ein System (1) zum Einführen einer Hand in eine Handschuhöffnung (21) eines geöffneten Einweghandschuhs (2) und Anziehen des Einweghandschuhs (2) an die Hand, dadurch gekennzeichnet, dass das System (1)
a) eine Apparatur (3) mit führbarem Spreizring (4) zum Öffnen des Einweghandschuhs (2), und
b) eine Haltevorrichtung (5) zur Aufnahme und zum Spannen von mindestens einem Einweghandschuh (2)
umfasst, wobei die Haltevorrichtung (5) in der Apparatur (3) angeordnet ist oder reversibel angeordnet werden kann und mindestens einen Einweghandschuh (2) umfasst.

Beansprucht wird auch ein Verfahren zum Einführen einer Hand in einen geöffneten Einweghandschuh (2) und Anziehen des Einweghandschuhs (2) an die Hand mit dem System (1), sowie die Verwendung des Systems (1).

## Beschreibung

Die vorliegende Erfindung bezieht sich auf ein System und ein Verfahren zum Einführen einer Hand in einen geöffneten Einweghandschuh und zum Anziehen des Einweghandschuhs an die Hand, sowie die Verwendung des Systems.

Einweghandschuhe, auch Einmalhandschuhe genannten, sind Handschuhe, die nach einmaligem Tragen weggeworfen und entsorgt werden. Sie werden beispielsweise von Personen im Gesundheitswesen eingesetzt, um sich selbst wie auch die Patienten vor Infektionen zu schützen. Auch kommen sie in - beispielsweise chemischen und biotechnologischen - Laboratorien zum Einsatz, finden jedoch auch Anwendung in der Lebensmittelindustrie und beim Verkauf von Lebensmitteln, beim Färben von Haaren sowie bei Reinigungsarbeiten.

Das Anziehen der Handschuhe braucht jeweils Zeit und ist besonders dann kritisch, wenn die Handschuhe steril sein müssen. Aus diesem Grund gibt es in der neueren Zeit verschiedene Vorschläge für eine Vorrichtung, anhand welcher die Handschuhe automatisch an die Hände der entsprechenden Personen angezogen werden sollen. Dabei soll nicht nur Zeit gespart werden, sondern es soll vermieden werden, dass beim Anziehen der Handschuhe diese verschmutzt werden.

So schlägt die WO-A-2016/174672 eine Handschuhvorrichtung vor, die ein Handschuhfach, eine Handschuhhebevorrichtung, eine Handschuhöffnungsvorrichtung und eine Handschuhaufblasvorrichtung umfasst. Dabei enthält das Handschuhfach Handschuhe, die Handschuh-Hebevorrichtung ist dazu ausgelegt, Handschuhe einzeln aus dem Handschuhfach zu heben und die Handschuhöffnung über die Handschuh-Öffnungsvorrichtung einzuführen, die Handschuhhebevorrichtung umfasst eine vertikale Stange mit einer oder mehreren Befestigungsvorrichtungen am unteren Ende sowie vertikale und horizontale Aktivatoren, das Handschuhöffnungsgerät hat vier Spreizfinger, die sich jeweils mit Aktivatoren horizontal und vertikal bewegen können, und die Handschuh-Aufblasvorrichtung umfasst einen Dichtungsring, ein Luftrohr und einen Aktivator, der den Dichtungsring vor und zurück bewegen kann. Die vorgeschlagene Vorrichtung ist jedoch gross, unhandlich und die Mechatronik ist kompliziert und teuer herzustellen. Die Prozesssicherheit ist nicht immer gegeben und oft nur schwer zu erreichen. Zudem besteht die Gefahr, dass der Handschuh von der Hebevorrichtung beschädigt wird.

Es ist somit die Aufgabe der vorliegenden Erfindung, die Nachteile des Standes der Technik zu verhindern. Dabei soll die Handschuhvorrichtung insbesondere einfach sein, um die Fehleranfälligkeit und die Kosten zu reduzieren. Dabei ist es wichtig, dass jeder einzelne Handschuh verwendet werden kann, ohne dass er beschädigt wird oder in der Apparatur stecken bleibt. Auch soll die Handschuhvorrichtung kompakt und idealerweise portabel sein, dass die gleiche Vorrichtung an verschiedenen Orten verwendet werden kann.

Die Aufgabe konnte überraschenderweise gelöst werden mit einem System (1) zum Einführen einer Hand in eine Handschuhöffnung (21) eines geöffneten Einweghandschuhs (2) mit Handschuhöffnung (21) und Anziehen des Einweghandschuhs (2) an die Hand, wobei das System (1)
a) eine Apparatur (3) mit führbarem Spreizring (4) zum Öffnen des Einweghandschuhs (2), und
b) eine Haltevorrichtung (5) zur Aufnahme und zum Spannen von mindestens einem Einweghandschuh (2) mit Handschuhöffnung (21) umfasst, wobei die Haltevorrichtung (5) in der Apparatur (3) angeordnet ist oder reversibel angeordnet werden kann und mindestens einen Einweghandschuh (2) umfasst.

Beansprucht wird auch Verfahren zum Einführen einer Hand in einen geöffneten Einweghandschuh (2) und Anziehen des Einweghandschuhs (2) an die Hand mit dem erfindungsgemässen System (1), wobei
a) die Haltevorrichtung (5) mit mindestens einem gespannten Einweghandschuh (2) ausgerüstet wird, wobei die Haltevorrichtung (5) vor- oder nachher in der Apparatur (3) angeordnet wird,
b) der Spreizring (4) der Apparatur (3) in den mindestens einen in der Haltevorrichtung (5) gespannten Einweghandschuh (2) geführt wird, wodurch die Handschuhöffnung (21) geöffnet wird,
c) der geöffnete Handschuh (2) von der Haltevorrichtung (5) gelöst wird, und der Spreizring (4) mit dem geöffneten Handschuh (2) in eine Position gebracht wird, an welcher die Hand in den geöffneten Handschuh (2) eingefügt wird, und
d) der an der Hand angezogene Handschuh (2) vom Spreizring (4) gelöst wird und die Hand aus der Apparatur (3) entfernt.

Zudem wird auch die Verwendung des erfindungsgemässen Systems (1) zum Einführen mindestens einer Hand in einen geöffneten Einweghandschuh (2) und Anziehen des mindestens einen Einweghandschuhs (2) an eine Hand, insbesondere im Gesundheitswesen, in Spitälern, bei Arzt- und Zahnarztpraxen, in Laboratorien wie chemische, biotechnologische und/oder biologische Laboratorien, bei der Produktion, Verarbeitung und/oder beim Verkauf von Lebensmittels, in Grossküchen, Frisiersalons, insbesondere beim Färben von Haaren, in Tattoo-Studios sowie für Reinigungsarbeiten jeglicher Art beansprucht.

Das erfindungsgemässe System (1), das erfindungsgemässe Verfahren sowie die erfindungsgemässe Verwendung weist überraschenderweise viele Vorteile auf. Die Handschuhvorrichtung ist einfach konstruiert, wodurch die Fehleranfälligkeit drastisch reduziert wird. Durch den Einsatz des Spreizrings (4) und der Haltevorrichtung (5) zur Aufnahme und zum Spannen von Einweghandschuhen (2) mit Handschuhöffnung (21) kann jeder einzelne Handschuh verwendet werden, ohne dass er beschädigt wird oder in der Apparatur stecken bleibt. Zudem ist die Handschuhvorrichtung kompakt und idealerweise portabel, sodass die gleiche Vorrichtung auch als mobile Vorrichtung eingesetzt und somit an verschiedenen Orten verwendet werden kann.

### Das System (1)

Das erfindungsgemässe System (1) ist geeignet zum Einführen mindestens einer Hand in eine Handschuhöffnung (21) eines geöffneten Einweghandschuhs (2), Anziehen des Einweghandschuhs (2) an die Hand und anschliessend Entfernen der Hand mit dem angezogenen Einweghandschuh (2). Dabei umfasst das System (1)
a) eine Apparatur (3) mit führbarem Spreizring (4) zum Öffnen des Einweghandschuhs (2), und
b) eine Haltevorrichtung (5) zur Aufnahme und zum Spannen von mindestens einem Einweghandschuh (2).

Dabei ist die Haltevorrichtung (5) in der Apparatur (3) angeordnet, oder die Haltevorrichtung (5) kann in der Apparatur (3) reversibel angeordnet werden. Die Haltevorrichtung (5) umfasst mindestens einen Einweghandschuh (2), bevorzugt eine Vielzahl, d.h. mindestens zwei, insbesondere 5 bis 100, ganz besonders bevorzugt 10 bis 60, Einweghandschuhe (2).

In einer bevorzugten Ausführungsform weist das System (1) mindestens zwei nebeneinander angeordnete Apparaturen (3) und Haltevorrichtungen (5) auf, wodurch mindestens zwei Hände miteinander, insbesondere beide Hände einer Person, in je einen Einweghandschuh (2) eingeführt und angezogen werden können.

Geeignete Einweghandschuhe (2), d.h. Handschuhe (2), sind im Handel erhältlich und umfassen Einweghandschuhe aus Latex, insbesondere aus Natur-Latex, Nitril, Vinyl und Polyethylen.

Dabei ist es oft vorteilhaft, wenn die Handschuhe (2) steril sind. Dazu können sie beispielsweise in der Haltevorrichtung (5) steril verpackt in die Apparatur (3) eingebracht werden. Bei Bedarf kann die Apparatur (3) - bevorzugt in Form einer verschliessbaren Box (7) - zusätzliche sterilisierende Vorrichtungen aufweisen, um mögliche Keime abzutöten.

Die Apparatur (3) mit führbarem Spreizring (4) des erfindungsgemässen Systems (1) ist geeignet zum Öffnen eines in der Haltevorrichtung (5) gespannten Einweghandschuhs (2) mit dem Spreizring (4), wodurch die Handschuhöffnung (21) des Einweghandschuhs (2) um den Spreizring (4) angeordnet wird, zur Entnahme des Einweghandschuhs (2) aus der Haltevorrichtung (5) mit dem Spreizring (4), sowie zum Führen des Spreizrings (4) mit dem geöffneten Handschuh (2) an eine Position, an welcher die Hand einfach und bequem durch die Handschuhöffnung (21), und somit durch den Spreizring (4), in den geöffneten Handschuh (2) eingeführt werden kann. Anschliessend kann der Handschuh (2) vom Spreizring (4) entfernt werden, beispielweise indem mit der Hand der Handschuh (2) aus dem Spreizring (4) gestossen wird, und die Hand mit dem angezogenen Handschuh (2) aus der Apparatur (3) herausgezogen werden.

Die Apparatur (3) umfasst den Spreizring (4) und, sofern vorhanden, die Bewegungsvorrichtung (6), die verschliessbare Box (7) sowie den Inkrement-Mechanismus (9).

Der Spreizring (4) ist führbar, d.h. er kann bewegt werden. Der Spreizring (4) kann, typischerweise mit Hilfe der Bewegungsvorrichtung (6), in den in der gespannten Haltevorrichtung (5) befindlichen Einweghandschuh (2) eingeführt werden, sodass der Handschuh (2) um den Spreizring (4) angeordnet und dadurch gespreizt wird. Dabei weist der Spreizring (4) eine so grosse Öffnung auf, dass eine Hand bequem durch den Spreizring (4) eingeführt und wieder herausgeführt werden kann. Stellt die Haltevorrichtung (5) eine Haltevorrichtung (51) dar, wird der Spreizring (4) immer an die gleiche Stelle geführt, wobei sich die Position der einzelnen Handschuhe (2) ändert. Stellt die Haltevorrichtung (5) eine Haltevorrichtung (52) dar, wird der Spreizring (4) für jede Entnahme eines Handschuhs (2) an einen anderen Ort geführt, da sich die Position der Handschuhe (2) nicht ändert.

Die Apparatur (3) umfasst bevorzugt eine Bewegungsvorrichtung (6), an welcher der Spreizring (4) angebracht ist, wobei die Bewegungsvorrichtung (6)
- den Spreizring (4) in einen in der Haltevorrichtung (5) gespannten Einweghandschuh (2) führen kann, wodurch der Handschuh (2) über einen Randbereich (41) des Spreizrings (4) angeordnet wird und sich der so durch den Spreizring (4) geöffnete Handschuh (2) von der Haltevorrichtung (5) löst und somit der Handschuh (2) durch den Spreizring (4) von der Haltevorrichtung (5) entnommen wird, und
- den Spreizring (4) mit dem daran angeordneten und gespannten Handschuh (2) in eine Position bringen kann, an welcher die Hand in den geöffneten Einweghandschuh (2) eingefügt werden kann,
wobei der Spreizring (4) in der Bewegungsvorrichtung (6) bevorzugt in einer S-Form geführt wird.

Die Bewegungsvorrichtung (6) ist - sofern vorhanden - Teil der Apparatur (3) und typischerweise an dieser angebracht. Die Bewegungsvorrichtung (6) kann manuell oder automatisch - beispielsweise mittels Lichtschranken oder durch Betätigen eines Fusspedals - betätigt werden.

Die Bewegungsvorrichtung (6) führt den Spreizring (4) zur Handschuhöffnung (21) des Handschuhs (2) und führt den Spreizring (4) in die Öffnung hinein. Durch eine geeignete Drehbewegung des Spreizrings (4) mittels Bewegungsvorrichtung (6) wird die Handschuhöffnung (21) um den Randbereich (41) des Spreizrings (4) gelegt, wodurch der Handschuh (2) sich von der Haltevorrichtung (5) löst. Anschliessend wird der Spreizring (4) zur Position geleitet, an welcher die Hand in den Handschuh eingeführt wird. Weist die Apparatur eine verschliessbare Box (7) mit mindestens einer Öffnung (71) auf, wird der Spreizring (4) zu dieser Öffnung (71) geleitet. Dabei wird - sofern mittels Vakuumpumpe (72) in der Box ein Unterdruck erstellt werden soll - zwischen dem Spreizring (4) und der Öffnung (71) eine geeignete Dichtung angeordnet, damit keine Aussenluft in die Box dringen kann.

In einer bevorzugten Ausführungsform des System (1) weist die Apparatur (3) eine verschliessbare Box (7) mit mindestens einer Öffnung (71) auf, wobei die Haltevorrichtung (5) - gegebenenfalls reversibel - in der Box (7) angeordnet ist und der Spreizring (4), bevorzugt mittels Bewegungsvorrichtung (6), zur Öffnung (71) geführt werden kann, wobei die Apparatur (3) eine Vakuumpumpe (72), d.h. Gasansaugpumpe, aufweist, mit welcher in der Box (7) bei geschlossener Öffnung (71) ein Unterdruck hergestellt werden kann. Dabei sind vorteilhafterweise die Öffnung (71) und der Spreizring (4) in deren Grösse und Form aufeinander angepasst, sodass sie optimal aufeinanderpassen, d.h. dass der Spreizring (4) die Öffnung (71) ganz überdecken kann. Dabei ist an der Öffnung (71) und/oder am Spreizring (4) bevorzugt eine geeignete Dichtung angebracht, um die Grenzfläche der Öffnung (71) zum Spreizring (4) optimal abzudichten. Ist nun am Spreizring (4) ein Handschuh (2) angeordnet und überdeckt der Spreizring (4) die Öffnung (71), kann im Innenraum der verschliessbaren Box (7) mit der Vakuumpumpe (72) Luft abgesaugt werden, wodurch ein Unterdruck entsteht. Dies wiederum führt dazu, dass die Aussenluft den am Spreizring (4) befestigte Handschuh (2) füllt, wodurch sich dieser aufbläst. Dadurch kann eine Hand bequem durch die Öffnung (71) und durch den Spreizring (4) in den aufgeblasenen Handschuh (2) eingeführt werden.

Umfasst die Apparatur (3) eine verschliessbare Box (7) und eine Vakuumpumpe (72), kann es vorteilhaft sein, wenn die Apparatur (3) auch mindestens eine Luftansaugglocke (73) umfasst, wobei die Luftansaugglocke (73) mit der Vakuumpumpe (72) verbunden ist und mindestens einen - aufgrund des in der Box (7) entstehenden Unterdrucks - mit Luft gefüllten Handschuh (2) aufnehmen kann. Wird nun von der Box (7) mit der Vakuumpumpe (72) Luft durch die Luftansaugglocken (73) abgesaugt, entsteht im Bereich des am Spreizring (4) angebrachten Handschuhs (2) lokal ein grösserer Unterdruck, wodurch der Handschuh (2) in die Luftansaugglocke (73) eingesogen und - aufgrund des grösseren Aussendrucks - aufgeblasen wird. Dadurch muss nicht der Druck des ganzen Boxvolumens gleich stark reduziert werden, sondern es genügt, lokal einen geeigneten Unterdruck herzustellen.

Die Haltevorrichtung (5) des erfindungsgemässen Systems (1) ist geeignet zur Aufnahme und zum Spannen von mindestens einem Einweghandschuh (2) und umfasst mindestens einen Einweghandschuh (2). Dadurch kann der führbare Spreizring (4) in die Handschuhöffnung (21) des gespannten Einweghandschuhs (2) geführt werden, wodurch der Einweghandschuh (2) geöffnet wird, von der Haltevorrichtung (5) entnommen und die Hand in den Handschuh (2) eingefügt werden.

Die Haltevorrichtung (5) ist in der Apparatur (3) angeordnet oder kann in der Apparatur (3) reversibel angeordnet werden. Einweghandschuhe (2) können an der Haltevorrichtung (5) in der Apparatur (3) angebracht werden. Alternativ - und oft bevorzugt - wird die Haltevorrichtung (5) ausserhalb der Apparatur (3) mit mindestens einem Handschuh (2) bestückt. Erfolgt das Bestücken der Haltevorrichtung (5) mit Handschuhen (2) ausserhalb der Apparatur (3), wird diese Tätigkeit bevorzugt durch Dritte durchgeführt, wodurch beispielsweise mit Handschuhen (2) bestückte Haltevorrichtungen (5) von Dritten hergestellt und zum Kauf angeboten werden können. Für den Einsatz des Systems (1) wird in diesem Fall die Haltevorrichtung (5) mit mindestens einem gespannten Handschuh (2) in die Apparatur (3) eingesetzt. Nach Entnahme des letzten Handschuhs (2) wird die Haltevorrichtung (5) aus der Apparatur (3) entnommen und entweder neu bestückt oder eine neue Haltevorrichtung (5) mit neuen Handschuhen (2) in die Apparatur (3) eingesetzt.

In einer bevorzugten Ausführungsform des System (1) weist die Haltevorrichtung (5) zwei zueinander parallel angeordnete Aufnahmevorrichtungen (8) zur Aufnahme einer Vielzahl an Einweghandschuhen (2) auf und an jeder Aufnahmevorrichtung (8) ist eine Vielzahl an Fixiermitteln (81) angeordnet, wobei die Einweghandschuhe (2) mittels der Fixiermittel (81) zwischen die Aufnahmevorrichtungen (8) gespannt werden können. Wird nun der Spreizring (4) in die Handschuhöffnung (21) eines gespannten Handschuhs (2) eingeführt, können durch eine geeignete Vorrichtung beim Spreizring (4) die Fixiermittel (81) vorteilhafterweise so bewegt werden, dass die Fixierung des Handschuhs (2) gelöst wird und die Handschuhöffnung (21) vollständig über dem Spreizring (4) angeordnet wird.

In einer bevorzugten Ausführungsform des Systems (1)
i) umfasst der Spreizring (4) seitliche Anordnungen (42), wobei, wenn der Handschuh (2) über den Randbereich (41) des Spreizrings (4) angeordnet wird, die seitlichen Anordnungen (42) auf die Fixiermittel (81), an denen der zu lösende Handschuh (2) fixiert ist, so einwirken können, dass sich der Handschuh (2) von den Fixiermitteln (81) und somit von der Haltevorrichtung (5) löst und der Handschuh (2) durch den Spreizring (4) von der Haltevorrichtung (5) entnommen werden kann, und/oder
ii) sind die Fixiermittel (81) an der Aufnahmevorrichtungen (8) so angebracht, dass die seitlichen Anordnungen (42) des Spreizrings (4) auf die Fixiermittel (81) einwirken können, wobei die Fixiermittel (81) bevorzugt in Form
   - einer Klemme vorliegen in welche der Handschuh (2) reversibel eingeklemmt sein kann, wobei die Aufnahmevorrichtungen (8) bevorzugt flächige Aufnahmevorrichtungen darstellen, wobei die Fixiermittel (81) in Form einer Klemme besonders geeignet sind für den Einsatz in der Haltevorrichtung (51); oder
   - von um eine Achse drehbare Fixiermittel (81) vorliegen, wobei die Aufnahmevorrichtungen (8) bevorzugt zylindrische, stabförmige Aufnahmevorrichtungen darstellen, um welche sich die Fixiermittel (81) drehen können. Dabei sind die drehbaren Fixiermittel (81) besonders geeignet für den Einsatz in der Haltevorrichtung (52).

In einer weiteren bevorzugten Ausführungsform des Systems (1) umfasst die Apparatur (3) einen Inkrement-Mechanismus (9) und die Haltevorrichtung (5) kann eine Vielzahl an Einweghandschuhen (2) aufnehmen und spannen, wobei der Inkrement-Mechanismus (9)
a) ein Inkrement-Mechanismus (91) darstellt, welcher die Haltevorrichtung (5), die Aufnahmevorrichtungen (8) und/oder die daran angeordneten Handschuhe (2) nach jeder Handschuhentnahme durch den Spreizring (4) um ein Inkrement bewegen kann, sodass der nachfolgende Handschuh (2) wieder am gleichen Ort angeordnet wird wie der zuvor entnommene Handschuh (2), und/oder
b) ein Inkrement-Mechanismus (92) darstellt, welcher den Spreizring (4) und gegebenenfalls die Bewegungsvorrichtung (6) - beispielsweise mittels Computerprogramm und/oder Sensoren - um ein Inkrement so steuert, dass nach einer Handschuhentnahme ein an der Haltevorrichtung (5) angeordneter nächster Handschuh (2) durch den Spreizring (4) von der Haltevorrichtung (5) entnommen werden kann, d.h. beispielsweise derjenige Handschuh (2), welcher an den zuvor entnommene Handschuh (2) angrenzte,
wobei das Inkrement bevorzugt der Distanz von zwei benachbarten Handschuhen (2) entspricht. Somit entnimmt der Spreizring (4) einen Handschuh (2) mit dem Inkrement-Mechanismus (91) immer am gleichen Ort, während mit dem Inkrement-Mechanismus (92) die Haltevorrichtung (5) mit den gespannten Handschuhen (2) statisch am gleichen Ort bleibt.

Ist der Inkrement-Mechanismus (9) ein Inkrement-Mechanismus (91), ist die Haltevorrichtung (5) des Systems (1) bevorzugt eine Haltevorrichtung (51) umfassend zwei zueinander parallel angeordnete Aufnahmevorrichtungen (8) mit Fixiermitteln (81), mindestens einer Einrastvorrichtung (82) sowie einem Behälter (83) zur Aufnahme einer Vielzahl von Einweghandschuhen (2). Dabei ist der Inkrement-Mechanismus (91) an der Apparatur (3), beispielsweise mit der Box (7), angebracht und mit der Bewegungsvorrichtung (6) so verbunden, dass nach jeder Entnahme eines Handschuhs (2) durch den Spreizring (4) der Inkrement-Mechanismus (91) um ein Inkrement bewegt wird. Die Haltevorrichtung (51) wird nun so in der Apparatur (3) angebracht oder angeordnet, dass die Einrastvorrichtung (82) der Haltevorrichtung (51) so mit dem Inkrement-Mechanismus (91) der Apparatur verbunden wird, dass bei jeder Bewegung des Inkrement-Mechanismus (91) um ein Inkrement, die Bewegung auf die Einrastvorrichtung (82) und somit auf die Haltevorrichtung (51) übertragen wird. Dadurch werden auch die in der Haltevorrichtung (51) gespannten Handschuhe (2) mit dem entsprechenden Inkrement so bewegt, dass nach einer Handschuhentnahme der an der Haltevorrichtung (5) angeordneter nächste, d.h. nachfolgende, Handschuh (2) wieder am gleichen Ort zu liegen kommt wie der zuvor entnommene Handschuh (2). Dies erlaubt eine problemlose Entnahme des nachfolgenden Handschuhs (2) durch den Spreizring (4).

Ist der Inkrement-Mechanismus (9) ein Inkrement-Mechanismus (92), ist die Haltevorrichtung (5) des Systems (1) bevorzugt eine Haltevorrichtung (52), bei welcher die gespannten Handschuhe (2) statisch an den Aufnahmevorrichtungen (8) mit Fixiermitteln (81) reversibel befestigt sind. Dabei ist der Inkrement-Mechanismus (92) so mit der Bewegungsvorrichtung (6) verbunden, dass nach jeder Entnahme eines Handschuhs (2) die Bewegung des Spreizrings (4) so ändert, dass der Spreizring (4) den an der Haltevorrichtung (5) angeordnete nächste Handschuh (2) von der Haltevorrichtung (5) entnommen wird. Somit wird der Spreizring (4) für jede Handschuhentnahme an einen anderen Ort, d.h. zu einem Ort mit einem gespannten Handschuh (2), geführt. Dies erfolgt typischerweise, insbesondere bevorzugt, mittels Computerprogramm und/oder Sensoren.

Die Haltevorrichtung (5) des Systems (1) stellt bevorzugt
i) eine Haltevorrichtung (51) mit Einweghandschuhen (2) dar, umfassend zwei zueinander parallel angeordnete Aufnahmevorrichtungen (8) mit Fixiermitteln (81), mindestens einer Einrastvorrichtung (82) sowie einem Behälter (83) zur Aufnahme einer Vielzahl von Einweghandschuhen (2), wobei
   - die Aufnahmevorrichtung (8) und Fixiermittel (81) im Behälter (83) angeordnet sind und die Handschuhe (2) durch die Fixiermittel (81) zwischen den beiden Aufnahmevorrichtungen (8) gespannt sind; wobei die Aufnahmevorrichtungen (8) bevorzugt in Form von flächigen Aufnahmevorrichtungen vorliegen,
   - der Behälter (83) im Bereich der Handschuhöffnung (21) mindestens eines Handschuhs (2) offen ist,
   - die Einrastvorrichtung (82) an mindestens einem Wandbereich des Behälters (83) angeordnet ist, und
   - die Einrastvorrichtung (82) mit der Aufnahmevorrichtung (8) in Verbindung steht und mit dem Inkrement-Mechanismus (9) in Verbindung gebracht werden kann, wodurch bei Betätigung des Inkrement-Mechanismus (9) auch die Einrastvorrichtung (82) und somit die Aufnahmevorrichtung (8) betätigt wird, wobei der Inkrement-Mechanismus (9) bevorzugt ein Inkrement-Mechanismus (91) darstellt,
   wodurch die Haltevorrichtung (51) besonders für die Apparatur (3) mit einem Inkrement Mechanismus (91) geeignet ist,
   oder
ii) eine Haltevorrichtung (52) mit Einweghandschuhen (2) dar, umfassend zwei zueinander parallel angeordnete Aufnahmevorrichtungen (8) mit Fixiermitteln (81), wobei die Handschuhe (2) durch die Fixiermittel (81) zwischen den beiden Aufnahmevorrichtungen (8) gespannt sind und mindestens ein Abstandshalter (84) die beiden Aufnahmevorrichtungen (8) so voneinander trennt, dass die Handschuhe (2) zwischen den Aufnahmevorrichtungen (8) gespannt sind, wobei die Haltevorrichtung (52) besonders für die Apparatur (3) mit einem Inkrement Mechanismus (92) geeignet ist. In dieser Ausführung weisen die Aufnahmevorrichtungen (8) bevorzugt eine zylindrische Form auf.

Liegt die Haltevorrichtung (5) als Haltevorrichtung (51) vor, umfasst sie parallel angeordnete Aufnahmevorrichtungen (8) mit Fixiermitteln (81), mindestens eine, bevorzugt vier, Einrastvorrichtung (82) sowie einen Behälter (83) zur Aufnahme einer Vielzahl von Einweghandschuhen (2). Dabei ist die Einrastvorrichtung (82) - beispielsweise in Form eines Zahnrads - bevorzugt so in eine Seitenwand des Behälters integriert, sodass von ausserhalb des Behälters der Inkrement-Mechanismus (91) in die Einrastvorrichtung (82) greifen kann. Zudem weist die Einrastvorrichtung (82) im inneren Bereich des Behälters (83) bevorzugt eine Führung auf, in welche die Aufnahmevorrichtungen (8), an welchen die Fixiermittel (81) und die gespannten Handschuhe (2) befestigt sind, angeordnet werden können. Durch Bewegung des Inkrement-Mechanismus (91) wird so die Bewegung auf die Einrastvorrichtung (82) und weiter auf die Aufnahmevorrichtungen (8) übertragen, werden auch die gespannten Handschuhe (2) in der Haltevorrichtung (51) um das entsprechende Inkrement bewegt. Dabei sind die Aufnahmevorrichtungen (8) der Haltevorrichtung (51) bevorzugt flächige Aufnahmevorrichtungen, an welchen die Fixiermittel (81) so angebracht sind, dass die seitlichen Anordnungen (42) des Spreizrings (4) während der Entnahme eines Handschuhs (2) - beispielsweise aufgrund einer Drehung des Spreizrings (4) - auf die Fixiermittel (81) einwirken können. Dabei sind die Fixiermittel (81) beispielsweise in Form einer Klemme, in welche der Handschuh (2) reversibel eingeklemmt werden kann, und welche durch die seitlichen Anordnungen (42) während oder nach der Entnahme des Handschuhs (2) mittels Druck geöffnet werden können.

Die Haltevorrichtung (51) weist mindestens eine, bevorzugt zwei, insbesondere vier, Einrastvorrichtungen (82) auf. Weist die Haltevorrichtung (51) vier Einrastvorrichtungen (82) auf, sind diese an den - typischerweise längeren - seitlichen Wänden des Behälters (83) im Bereich der seitliche Kanten angeordnet. Dadurch dienen sie als Antrieb der Aufnahmevorrichtungen (8). In dem jeder Drehpunkt der Aufnahmevorrichtungen (8) angetrieben wird, wird so ein gleichmässiger Antrieb und Vortrieb der fixierten Handschuhe (2) gewährleistet. Weist die Haltevorrichtung (51) zwei Einrastvorrichtungen (82) auf, sind diese bevorzugt auf je einer seitlichen Wand des Behälters (83) angeordnet, um die beiden Aufnahmevorrichtungen (8) separat antreiben zu können. Weist die Haltevorrichtung (51) nur eine Einrastvorrichtung (82) auf, ist dies bevorzugt mittels einer Achse mit beispielsweise einer Rolle auf der gegenüberliegenden Seite des Behälters (83) verbunden. Werden weniger als 4 Einrastvorrichtungen (82) eingesetzt, werden diese typischerweise durch Rollen ersetzt.

Liegt die Haltevorrichtung (5) als Haltevorrichtung (52) vor, sind die Aufnahmevorrichtungen (8) bevorzugt in Form von zylindrischen, stabförmigen Aufnahmevorrichtungen, beispielsweise in Form von feinen Holz-, Kunststoff- oder Metallstangen mit einem Durchmesser von ca. 1 bis 5 mm. Die Fixiermittel (81) sind dabei bevorzugt so ausgestaltet, dass sie sich um eine Achse, d.h. um die stabförmige Aufnahmevorrichtung, drehen können. In Drehrichtung der Fixiermittel (81) weisen sie auf einer Seite beispielsweise eine Verdickung auf, mit welcher die Handschuhe (2) gespannt werden können, ohne sie zu beschädigen. Auf der anderen Seite des Drehpunkts weisen die Fixiermittel (81) bevorzugt eine Verlängerung auf. Wird nun der Spreizring (4) in die Handschuhöffnung (21) eingeführt und weiter gedreht um die ganze Handschuhöffnung (21) um den Spreizring (4) anzuordnen, drücken die seitlichen Anordnungen (42) des Spreizrings (4) auf die Verlängerungen des Fixiermittels (81), wodurch sich diese nach unten abdrehen, wodurch auf der anderen Seite des Drehpunkts die Verdickung des Fixiermittels (81) nach oben und aus der Handschuhöffnung (21) kippt. Somit löst sich der Handschuh (2) vom Fixiermittel (81) und er kann, um den Spreizring (4) gespannt, aus der Haltevorrichtung (5, 52) entnommen werden.

Die Haltevorrichtung (52) wird beispielsweise in die verschliessbare Box (7) so eingebracht, dass die beiden Aufnahmevorrichtungen (8) in einem Endbereich der Aufnahmevorrichtungen (8) an der Apparatur (3) - beispielsweise mittels Einstecken - befestigt werden. Dabei dienen diese an der Apparatur (3) fest angeordneten Steckverbindungen vorteilhafterweise auch als - bevorzugt zusätzliche - Abstandshalter (84). Dabei kann die Haltevorrichtung (52) an der Apparatur (3) - beispielsweise in der Box (7) - fest installiert sein und die Handschuhe (2) in der Apparatur (3) an der Haltevorrichtung (52) gespannt werden. Oft ist es jedoch vorteilhaft, wenn das Anbringen der Handschuhe (2) an der Haltevorrichtung (52) ausserhalb der Apparatur (3) erfolgt und die Haltevorrichtung (52) zusammen mit den gespannten Handschuhen (2) in die Apparatur eingeführt und dort befestigt wird.

Wenn die Haltevorrichtung (5, 52) an der Apparatur (3) angebracht ist, sind die Handschuhe (2) typischerweise statisch an der Aufnahmevorrichtungen (8) und somit an der Haltevorrichtung (5, 52) angeordnet, d.h. die Handschuhe (2) verändern ihre Position auch nach einer Handschuhentnahme nicht. Hingegen wird der Spreizring (4), typischerweise mittels Computerprogramm, zu einem gespannten Handschuh (2) geführt, wo er auch aus der Haltevorrichtung (5, 52) entnommen werden kann.

Ist die Haltevorrichtung (5, 51, 52) als Einweg-Haltevorrichtung (5, 51, 52) konzipiert, wird sie bevorzugt aus rezyklierbaren und/oder kompostierbaren Materialien hergestellt. Besonders geeignete Materialien umfassen Karton, Papier und Holz.

### Das Verfahren

Das erfindungsgemässe Verfahren eignet sich zum Einführen einer Hand in einen geöffneten Einweghandschuh (2) und zum Anziehen des Einweghandschuhs (2) an die Hand mit dem erfindungsgemässen System (1).

Mit dem Verfahren wird
a) die Haltevorrichtung (5) mit mindestens einem gespannten Einweghandschuh (2) ausgerüstet, wobei die Haltevorrichtung (5) vor- oder nachher in der Apparatur (3) angeordnet wird,
b) der Spreizring (4) der Apparatur (3) in den mindestens einen in der Haltevorrichtung (5) gespannten Einweghandschuh (2) geführt, wodurch die Handschuhöffnung (21) geöffnet wird,
c) der geöffnete Handschuh (2) von der Haltevorrichtung (5) gelöst, und der Spreizring (4) mit dem geöffneten Handschuh (2) in eine Position gebracht, an welcher die Hand in den geöffneten Handschuh (2) eingefügt wird, und
d) der an der Hand angezogene Handschuh (2) vom Spreizring (4) gelöst und die Hand aus der Apparatur (3) entfernt.

Ist die Haltevorrichtung (5) eine Haltevorrichtung (51), wird die Haltevorrichtung (51) im Verfahrensschritt a) bevorzugt ausserhalb der Apparatur (3) mit mindestens einem Handschuh (2) ausgerüstet und anschliessend die Haltevorrichtung (51) zusammen mit mindestens einem Handschuh (2), bevorzugt einer Vielzahl an Handschuhen (2), in die Apparatur (3) eingefügt.

Ist die Haltevorrichtung (5) eine Haltevorrichtung (52), kann die Haltevorrichtung (5) im Verfahrensschritt a) analog der Haltevorrichtung (51) in die Apparatur (3) eingefügt werden. Alternativ ist die Haltevorrichtung (52) in der Apparatur (3) fest installiert und die Handschuhe (2) werden in der Apparatur (3) an der Haltevorrichtung (52) angebracht. Dabei werden die Handschuhe (2) an den Fixiermitteln (81) befestigt und zwischen den beiden Aufnahmevorrichtungen (8), an welchen die Fixiermittel (81) angebracht sind, gespannt. Der mindestens eine Abstandshalter (84), welcher die beiden Aufnahmevorrichtungen (8) voneinander trennt, gewährt, dass die Handschuhe (2) gespannt bleiben. In dieser Ausführungsform sind die Aufnahmevorrichtungen (8) der Haltevorrichtung (52) bevorzugt zylindrische, stabförmige Aufnahmevorrichtungen, beispielsweise in Form von Metallstangen, und die Fixiermittel (81) bevorzugt drehbare Fixiermittel (81), die sich um eine Achse in Form der stabförmigen Aufnahmevorrichtung drehen können.

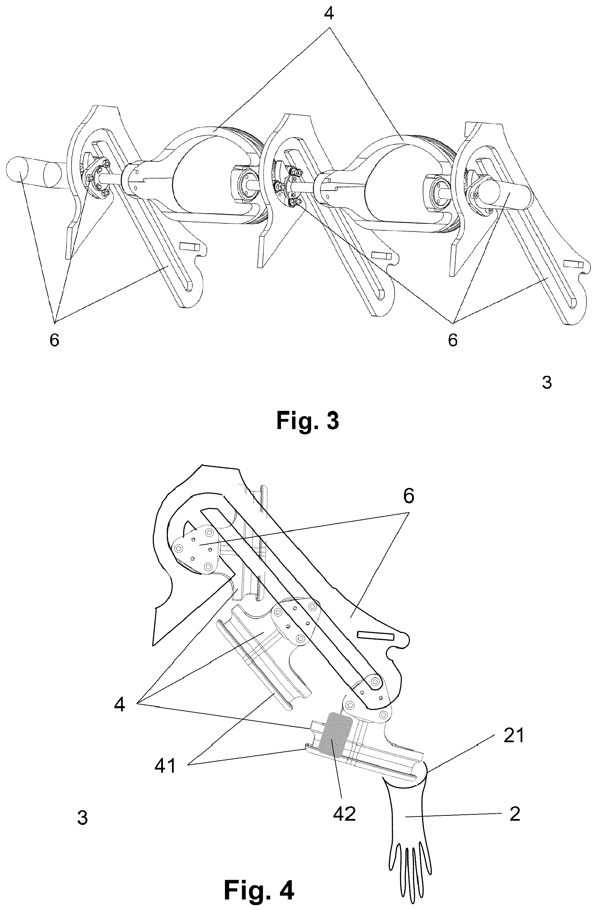

In einer bevorzugten Ausführungsform des erfindungsgemässen Verfahrens wird der Spreizring (4) an der Bewegungsvorrichtung (6) angebracht, wobei die Bewegungsvorrichtung (6)
- den Spreizring (4) in die Handschuhöffnung (21) eines in der Haltevorrichtung (5) gespannten Einweghandschuhs (2) führt, wodurch die Handschuhöffnung (21) über den Randbereich (41) des Spreizrings (4) angeordnet wird und sich der so durch den Spreizring (4) geöffnete Handschuh (2) von der Haltevorrichtung (5) löst und der Handschuh (2) durch den Spreizring (4) von der Haltevorrichtung (5) entnommen wird, und
- den Spreizring (4) mit dem daran angeordneten und gespannten Handschuh (2) in eine Position, insbesondere an die Öffnung (71), bringt, an welcher die Hand in den geöffneten Einweghandschuh (2) eingefügt werden kann,
wobei die Bewegungsvorrichtung (6) mit dem Spreizring (4) bevorzugt in einer S-Form geführt wird. Eine nicht-limitierende Ausführungsform ist in Fig. 4 dargestellt.

In einer anderen bevorzugten Ausführungsform des Verfahrens
i) umfasst der Spreizring (4) seitliche Anordnungen (42), wobei, wenn der Handschuh (2) über den Randbereich (41) des Spreizrings (4) angeordnet wird, die seitlichen Anordnungen (42) auf die Fixiermittel (81), an denen der zu lösende Handschuh (2) fixiert ist, so einwirken, dass sich der Handschuh (2) von den Fixiermitteln (81) und somit von der Haltevorrichtung (5) löst und der Handschuh (2) durch den Spreizring (4) von der Haltevorrichtung (5) entnommen wird, und/oder
ii) werden die Fixiermittel (81) an der Aufnahmevorrichtungen (8) so angebracht, dass die seitlichen Anordnungen (42) des Spreizrings (4) bei der Entnahme eines Handschuhs (2) auf die Fixiermittel (81) so einwirken, dass sich der Handschuh (2) von den Fixiermitteln (81) löst, wobei die Fixiermittel (81) bevorzugt in Form
   - einer Klemme vorliegen in welche der Handschuh (2) reversibel eingeklemmt sein kann, wobei die Aufnahmevorrichtungen (8) bevorzugt flächige Aufnahmevorrichtungen darstellen, wobei die Fixiermittel (81) in Form einer Klemme besonders geeignet sind für den Einsatz in der Haltevorrichtung (51); oder
   - von um eine Achse drehbare Fixiermittel (81) vorliegen, wobei die Aufnahmevorrichtungen (8) bevorzugt zylindrische, stabförmige Aufnahmevorrichtungen darstellen, um welche sich die Fixiermittel (81) drehen können. Dabei sind die drehbaren Fixiermittel (81) besonders geeignet für den Einsatz in der Haltevorrichtung (52).

In einer weiteren bevorzugten Ausführungsform des Verfahrens
- führt die Bewegungsvorrichtung (6) den Spreizring (4) mit dem am Spreizring (4) angeordneten und geöffneten Handschuh (2) an die Öffnung (71) der verschliessbaren Box (7) heran, sodass die vom Handschuh (2) abgewandte Seite des Spreizrings (4) die Öffnung (71) umschliesst, wodurch der Handschuh (2) die Abgrenzung zwischen Innenbereich und Aussenbereich der Box (7) darstellt,
- wird mittels Vakuumpumpe (72), d.h. Gasansaugpumpe, gegebenenfalls über die Luftansaugglocke (73), in der Box (7) ein Unterdruck erzeugt, wodurch der Handschuh (2) nach innen, gegebenenfalls in die Luftansaugglocke (73), gesogen wird, wodurch sich der Handschuh (2) vollständig öffnet,
- wird von der Aussenseite der Box (7) die Hand durch die Öffnung (71) in den geöffneten Handschuh (2) eingefügt, und
- wird die Vakuumpumpe (72) gestoppt, der Handschuh (2) vom Spreizring (4) gelöst und die Hand mit dem angezogenen Handschuh (2) aus der Box (7) entfernt.

Dabei ist es oft vorteilhaft, wenn eine Dichtung zwischen dem Spreizring (4) und der Öffnung (71) angeordnet ist, um zu verhindern, dass bei der Betätigung der Vakuumpumpe (72) Aussenluft in die Box (7) gelangen kann.

In einer bevorzugten Ausführungsform des Verfahrens umfasst die Apparatur (3)
i) einen Inkrement-Mechanismus (91) und die Haltevorrichtung (5) eine Haltevorrichtung (51) mit einer Einrastvorrichtung (82) darstellt, wobei die Haltevorrichtung (51) so in die Apparatur (3) eingebracht wird, dass die Einrastvorrichtung (82) mit dem Inkrement-Mechanismus (91) verbunden wird, wodurch sich durch Betätigung des Inkrement-Mechanismus (91) die Position der Handschuhe (2) in der Haltevorrichtung (51) verändert, oder
ii) einen Inkrement-Mechanismus (92) umfasst und die Haltevorrichtung (5) eine Haltevorrichtung (52) darstellt, wobei der Inkrement-Mechanismus (92), gegebenenfalls über die Bewegungsvorrichtung (6), mit dem Spreizring (4) verbunden wird, wodurch sich die Position des Spreizrings (4), typischerweise mittels Computerprogramm, bei jeder Entnahme eines Handschuhs (2) verändert. Dabei sind die Handschuhe (2) typischerweise statisch an der Aufnahmevorrichtungen (8) und somit an der Haltevorrichtung (5, 52) angeordnet und verändern bei einer Handschuhentnahme ihre Position nicht.

### Die Verwendung

Das erfindungsgemässe System (1) und das erfindungsgemässe Verfahren können vielseitig verwendet werden.

Eine ganz bevorzugte Verwendung umfasst das Einführen mindestens einer Hand in einen geöffneten Einweghandschuh (2) und das Anziehen des mindestens einen Einweghandschuhs (2) an eine Hand, insbesondere im Gesundheitswesen, in Spitälern, bei Arzt- und Zahnarztpraxen, in Laboratorien wie chemische, biotechnologische und/oder biologische Laboratorien, bei der Produktion, Verarbeitung und/oder beim Verkauf von Lebensmittels, in Grossküchen, Frisiersalons, insbesondere beim Färben von Haaren, in Tattoo-Studios sowie für Reinigungsarbeiten jeglicher Art.

Es werden folgende Bezugszeichen verwendet:
1. System (1)
2. Einweghandschuh (2)
   21 mit Handschuhöffnung (21)
3. Apparatur (3)
4. Spreizring (4)
   41 Randbereich (41) des Spreizrings (4)
   42 zwei seitliche Anordnungen (42) des Spreizrings (4)
5. Haltevorrichtung (5)
   51 Haltevorrichtung (51) umfassend zwei zueinander parallel angeordnete Aufnahmevorrichtungen (8) mit Fixiermitteln (81), mindestens einer Einrastvorrichtung (82) sowie einen Behälter (83) zur Aufnahme einer Vielzahl von Einweghandschuhen (2)
   52 Haltevorrichtung (52) bei welcher die Handschuhe (2) durch die Fixiermittel (81) zwischen den beiden Aufnahmevorrichtungen (8) gespannt sind
6. Bewegungsvorrichtung (6)
7. verschliessbare Box (7)
   71 mindestens eine Öffnung (71) der Box (7)
   72 Vakuumpumpe (72) um in der Box (7) einen Unterdruck herzustellen
   73 Luftansaugglocke (73) und mindestens einen mit Luft gefüllten Handschuh (2) aufzunehmen
8 Aufnahmevorrichtungen (8) zur Aufnahme einer Vielzahl an Einweghandschuhen (2)
   81 Fixiermittel (81) um die Einweghandschuhe (2) zwischen den Aufnahmevorrichtungen (8) zu spannen
   82 Einrastvorrichtung (82)
   83 Behälter (83) mit einer Vielzahl an Einweghandschuhen (2)
   84 Abstandshalter (84)
9 Inkrement-Mechanismus (9)
   91 Inkrement-Mechanismus (91), welcher die Haltevorrichtung (5), die Aufnahmevorrichtungen (8) und/oder die daran angeordneten Handschuhe (2) um ein Inkrement bewegen kann
   92 Inkrement-Mechanismus (92), welcher die Bewegungsvorrichtung (6) steuert

Im Folgenden werden nicht-limitierende, bevorzugte Ausführungsformen des erfindungsgemässen Systems (1) mit der Apparatur (3) mit dem führbaren Spreizring (4), der Haltevorrichtung (5) sowie dem erfindungsgemässen Verfahren anhand der nachfolgenden Zeichnungen beschrieben. Diese sind nicht einschränkend auszulegen und werden als Bestandteil der Beschreibung verstanden:

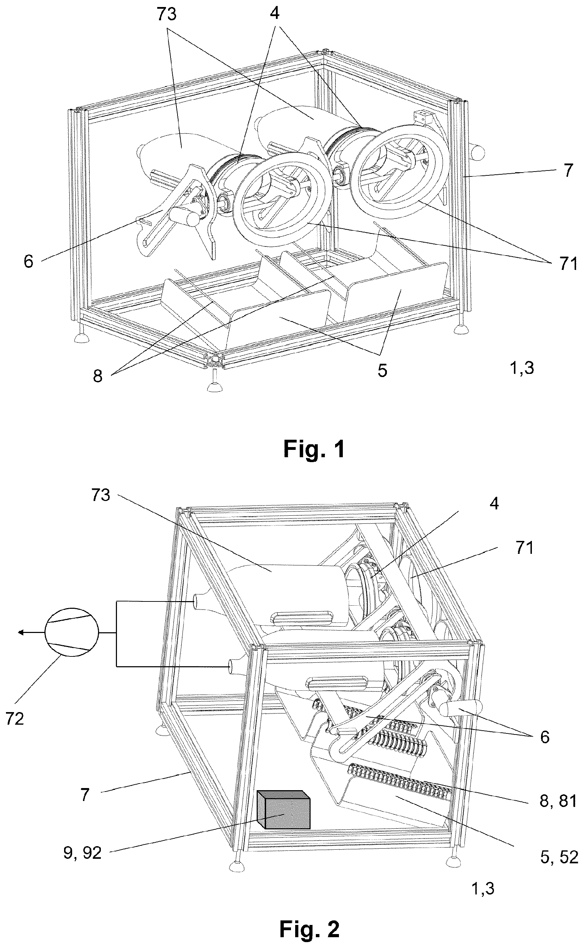

- Fig. 1: zeigt das erfindungsgemässe System (1) mit der Apparatur (3) und dem führbaren Spreizring (4), sowie der Haltevorrichtung (5). Die Apparatur ist in einer verschliessbaren Box (7) angeordnet, wobei die Rahmenprofile zur vorderen oberen Ecke zur besseren Übersicht weggelassen wurden. Die Box (7) weist beispielhaft zwei Öffnungen (71) auf, in welche beide Hände simultan hineingeschoben werden können, um an beide Hände gleichzeitig einen Einweghandschuh (2) anzuziehen. Im unteren Bereich der Box (7) sind schemenhaft zwei Haltevorrichtungen (5) mit stabartigen Aufnahmevorrichtungen (8) zur Aufnahme einer Vielzahl von Handschuhen (2) angezeigt.
Auf den beiden Aussenseiten der Box (7) auf Höhe der Öffnungen (71) ist beispielhaft eine manuelle Bewegungsvorrichtung (6) angeordnet. Mit einem Handgriff, welcher mit dem Spreizring (4) verbunden und Teil der Bewegungsvorrichtung (6) ist, kann der Spreizring (4) von der Öffnung (71) zu einem geöffneten Handschuh (2) (nicht dargestellt) geführt werden.
Hinter dem Spreizring (4) - von der Öffnung (71) aus betrachtet - ist beispielhaft eine Luftansaugglocke (73) angeordnet.
- Fig. 2: zeigt das erfindungsgemässe System (1) mit der Apparatur (3) und dem führbaren Spreizring (4), sowie der Haltevorrichtung (5) in Form der Haltevorrichtung (52). Die Apparatur ist in einer verschliessbaren Box (7) angeordnet, welche durch die einzelnen Rahmenprofile angezeigt ist.
Im unteren Bereich der Box (7) sind schemenhaft zwei Haltevorrichtungen (5) mit stabartigen Aufnahmevorrichtungen (8) und darin angebrachten Fixiermitteln (81) zum Fixieren und Spannen einer Vielzahl von Handschuhen (2) angezeigt.
Anhand der Befestigungsvorrichtung (6), die mit der Box (7) verbunden ist, kann der Spreizring (4) beispielhaft mit dem Handgriff in Richtung Haltevorrichtung (5) geführt werden. Dabei entsteht eine S-förmige Bewegung des Handgriffs und des Spreizrings (4), wodurch sich dieser neigt und optimal in die Handschuhöffnung (21) eines gespannten, geöffneten Handschuhs (2) (nicht dargestellt) geführt wird. Am Schluss der Bewegung dreht sich der Spreizring (4) erneut, wodurch sich die Handschuhöffnung (21) ganz um den Spreizring (4) anordnet. Dies erlaubt die Entfernung des Handschuhs (2) von der Haltevorrichtung, beispielsweise indem seitliche Anordnungen (42) am Spreizring (4) (nicht dargestellt) einen Druck auf die Fixiermittel (81) ausüben, wodurch der Handschuh (2) sich vom Fixiermittel (81) löst. Die Befestigungsvorrichtung (6) ist mit dem Inkrement-Mechanismus (9, 92) verbunden, welcher - typischerweise mittels Computerprogramm - bei jeder Entnahme eines Handschuhs die Position des Spreizrings (4) für die Handschuhentnahme verändert. So sind die Handschuhe (2) typischerweise statisch an der Aufnahmevorrichtungen (8) und somit an der Haltevorrichtung (5, 52) angeordnet und verändern bei einer Handschuhentnahme ihre Position nicht.
Hinter den Spreizringen (4) - von den Öffnungen (71) betrachtet - sind die Luftansaugglocken (73) angeordnet, welche einen mit Luft gefüllten Handschuh (2) aufnehmen können. Die Luftansaugglocken (73) sind mit der Vakuumpumpe (72), d.h. einer Gasansaugpumpe, verbunden, um in der verschliessbaren Box (7) einen Unterdruck herzustellen. Wird nun von der Box (7) Luft durch die Luftansaugglocken (73) abgesaugt, entsteht im Bereich des am Spreizring (4) angebrachten Handschuhs (2) lokal ein grösserer Unterdruck, wodurch der Handschuh (2) in die Luftansaugglocke (73) eingesogen und - aufgrund des grösseren Aussendrucks - aufgeblasen wird.
- Fig. 3: zeigt beispielhaft die Apparatur (3) umfassend die beiden Spreizringe (4) des in Fig. 1 und Fig. 2 dargestellten Systems (1) mit der Bewegungsvorrichtung (6).
- Fig. 4: zeigt beispielhaft die Bewegungsvorrichtung (6) mit drei an unterschiedlichen Positionen dargestellten Spreizringe (4). Der oberste Spreizring (4) ist in der Ausgangsposition und - mit geeigneter, nicht dargestellter Dichtung - grenzt beispielsweise an eine Öffnung (71) der Box (7) (nicht dargestellt). Wird nun der Spreizring (4) in der Bewegungsvorrichtung (6) nach unten geführt, dreht sich der Spreizring (4), sodass der zunächst oberste Punkt zum untersten Punkt des Spreizring (4) wird. In dieser Position kann er auch in eine schlanke Handschuhöffnung (21) eingeführt werden. An diesem Punkt - dies zeigt die dritte, unterste Position des Spreizrings (4) - dreht sich der Spreizring (4), nachdem er mit einem Teil in die Handschuhöffnung (21) eingefahren ist. Durch dies Drehung kann der Spreizring (4) die ganze Handschuhöffnung (21) umfassen und anschliessend von der Haltevorrichtung (5) lösen. Am Spreizring (4) in der untersten Position ist beispielhaft eine seitliche Anordnung (42) angebracht, mit welcher - aufgrund der Drehung des Spreizrings (4) nachdem der Handschuh (2) um den Spreizring (4) zu liegen kommt - einen Druck auf die Fixiermittel (81) ausgeübt wird, wodurch der Handschuh (2) von den Fixiermitteln (81) gelöst wird.

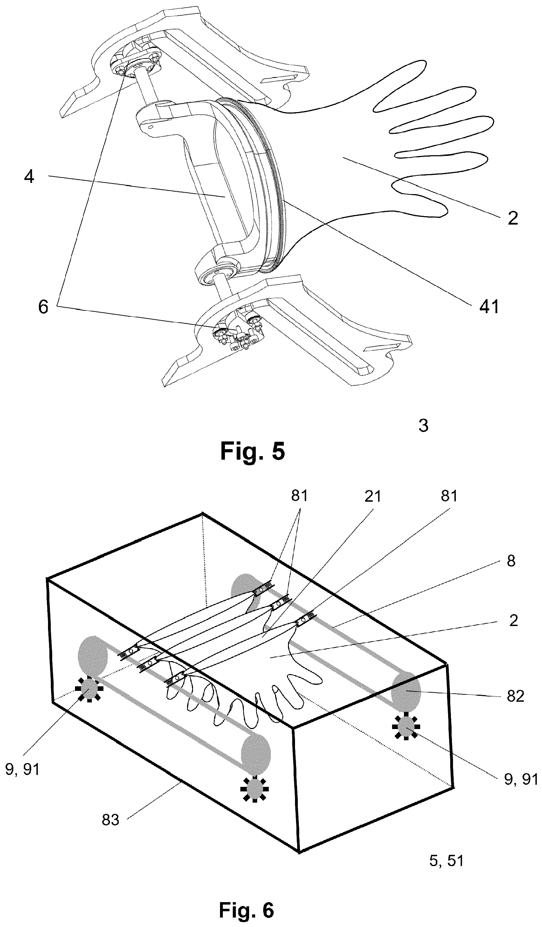

- Fig. 5: zeigt die Apparatur (3) mit dem Spreizring (4), welcher an der Bewegungsvorrichtung (6) befestigt ist. Über dem Randbereich (41) des Spreizrings (4) ist der Handschuh (2) angeordnet. Dadurch kann eine Hand bequem durch den Spreizring (4) in den Handschuh (2) eingeführt werden. Indem beispielsweise die Hand anschliessend noch weiter in den Handschuh (2) gestossen wird, löst sich dieser vom Spreizring (4), sodass die Hand mit dem angezogenen Handschuh (2) aus dem Spreizring (4) hinausgeführt werden kann.
- Fig. 6: zeigt eine perspektivische Ansicht der Haltevorrichtung (5) in Form einer beispielhaft dargestellten Haltevorrichtung (51) umfassend einen Behälter (83) zur Aufnahme einer Vielzahl von Einweghandschuhen (2), zwei zueinander parallel angeordnete, flächige Aufnahmevorrichtungen (8) mit einer Vielzahl von Fixiermitteln (81) und vier Einrastvorrichtungen (82). Die Aufnahmevorrichtungen (8) sind bevorzugt in Form eines Endlosbands um die Einrastvorrichtungen (82) angeordnet.
Die Einrastvorrichtungen (82) sind in den zwei zueinander parallelen längeren Seitenwänden so angeordnet, dass sie sich um die eigene Achse drehen können. Ein Teil der Einrastvorrichtung (82) befindet sich innerhalb des Behälters (83) und dient als Führung und Antrieb für die Aufnahmevorrichtungen (8) mit den Fixiermitteln (81). Dabei sind die Aufnahmevorrichtungen (8) bevorzugt flächige Aufnahmevorrichtungen und die Fixiermittel (81) liegen bevorzugt in Form einer Klemme vor, in welche der Handschuh (2) im Bereich der Handschuhöffnung (21) reversibel eingeklemmt und leicht gespannt werden kann. Der andere Teil der Einrastvorrichtungen (82) befindet sich ausserhalb des Behälters (83) und kann mit dem InkrementMechanismus (9, 91) der Apparatur (3) verbunden werden, beispielsweise mittels Einrasten.
Nach jeder Entnahme eines Handschuhs (2) wird nun der InkrementMechanismus (9, 91) - beispielsweise anhand eines mechanischen Mechanismus oder mittels Software-Steuerung - um ein Inkrement bewegt. Diese Bewegung überträgt sich auf die Einrastvorrichtung (82) und weiter auf die Aufnahmevorrichtungen (8) an welcher die Handschuhe (2) mittels Fixiermittel (81) gespannt sind. Somit sind die Haltevorrichtung (5, 51) und der Inkrement-Mechanismus (9, 91) derart ausgestaltet, dass nach einer Handschuhentnahme der an der Haltevorrichtung (5) angeordnete nächste Handschuh (2) - und somit alle Handschuhe (2) - durch den Spreizring (4) wieder am gleichen Ort entnommen werden kann wie der vorhergehende. Dabei ist der Behälter (83) zumindest im Bereich der Handschuhentnahme offen.

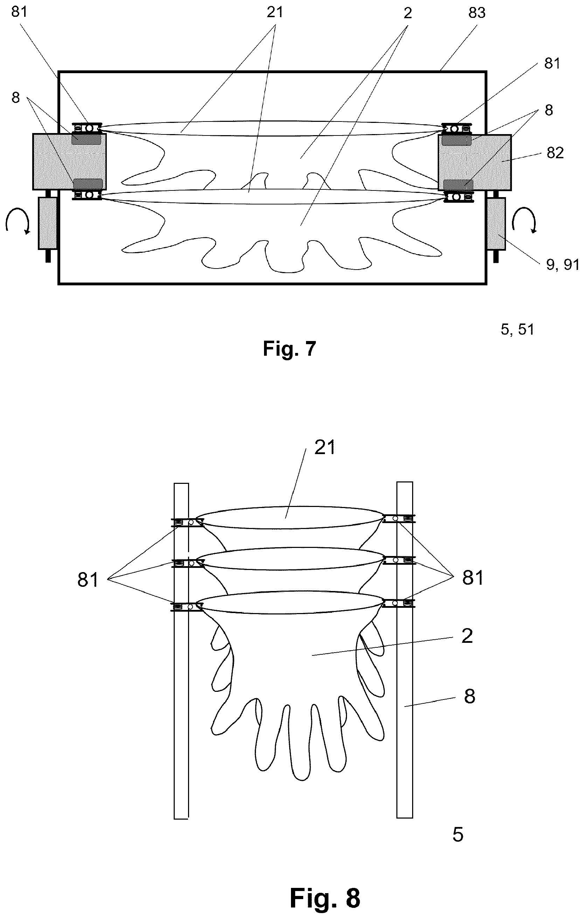

- Fig. 7: zeigt die Haltevorrichtung (5, 51) mit dem Behälter (83) und dem Inkrement-Mechanismus (9, 91) der Fig. 6, jedoch von der vorderen Seite. Auf beiden äusseren Seiten des Behälters (83) sind Einrastvorrichtungen (82) mit dem Inkrement-Mechanismus (9, 91) verbunden.
Im inneren Bereich des Behälters (83) sind die Aufnahmevorrichtungen (8) mit den Fixiermitteln (81) in Form von Klemmen um die Einrastvorrichtungen (82) angeordnet. Dabei werden die Handschuhe (2) durch die Fixiermittel (81) leicht gespannt, sodass die Handschuhöffnung (21) noch genügend gross ist, um den Spreizring (4) (nicht dargestellt) aufzunehmen. Dabei sind entlang der ganzen Aufnahmevorrichtung (8) Handschuhe (2) gespannt, wodurch die Handschuhe (2) 2-lagig im Behälter (83) angeordnet sein können. Dadurch können im Behälter (83) mehr Handschuhe (2) fixiert werden.
Wird nun der Spreizring (4) zum oberen Handschuh (2) geführt, und umfasst der Spreizring (4) seitliche Anordnungen (42), drücken diese - nachdem der Handschuh (2) über den Randbereich (41) des Spreizrings (4) gelegt wird - auf die Fixiermittel (81), wodurch sich die Klemmen, d.h. die Fixiermittel (81) lösen und der Handschuh (2) vom Behälter (83) und der Haltevorrichtung (5, 51) entnommen werden kann.
- Fig. 8: zeigt beispielhaft, wie Handschuhe (2) im Bereich der Handschuhöffnungen (21) an Fixiermitteln (81) gespannt werden können, wobei die Fixiermittel (81) an den Aufnahmevorrichtungen (8) angebracht sind. Dabei sind die Aufnahmevorrichtungen (8) bevorzugt flächige Aufnahmevorrichtungen und die Fixiermittel (81) liegen bevorzugt in Form von Klemmen vor.
Die so fixierten Handschuhe (2) werden typischerweise im Behälter (83) der Haltevorrichtung (5, 51) angebracht, indem die Aufnahmevorrichtungen (8) um die Einrastvorrichtungen (82) (siehe Fig. 6 und Fig. 7) gelegt und die Enden der Aufnahmevorrichtungen zu einem Endlosband verbunden werden.

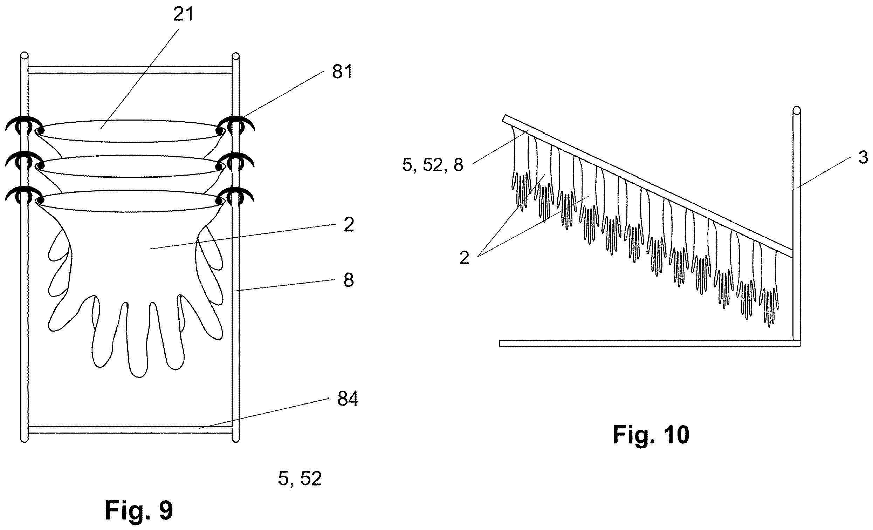

- Fig. 9: zeigt beispielhaft eine Haltevorrichtung (52) mit Einweghandschuhen (2). Die Haltevorrichtung (52) umfasst zwei zueinander parallel angeordnete Aufnahmevorrichtungen (8) mit mindestens einem Abstandshalter (84). Dieser gewährleistet, dass die Handschuhe (2) zwischen den Aufnahmevorrichtungen (8) gespannt sind.
Die Haltevorrichtung (52) wird beispielsweise in die verschliessbare Box so eingebracht, dass die beiden Aufnahmevorrichtungen (8) in einem Endbereich der Aufnahmevorrichtungen (8) an der Apparatur (3) - beispielsweise mittels Einstecken - befestigt werden. Dabei dienen diese an der Apparatur (3) fest angeordneten Steckverbindungen vorteilhafterweise auch als - bevorzugt zusätzlichen - Abstandshalter (84).
Die Aufnahmevorrichtungen (8) liegen bevorzugt in Form von zylindrischen, stabförmigen Aufnahmevorrichtungen, beispielsweise in Form von feinen Holz-, Kunststoff- oder Metallstangen vor. Dabei sind die Fixiermittel (81) bevorzugt so ausgestaltet, dass sie sich um eine Achse, d.h. um die stabförmige Aufnahmevorrichtung, drehen können. In Drehrichtung der Fixiermittel (81) weisen sie auf einer Seite beispielsweise eine Verdickung auf, welche es erlaubt, die Handschuhe (2) zu spannen, ohne dass sie beschädigt werden. Auf der anderen Seite des Drehpunkts weisen die Fixiermittel eine Verlängerung auf. Wird nun der Spreizring (4) (nicht dargestellt) in eine Handschuhöffnung (21) eingeführt und weiter gedreht um die ganze Handschuhöffnung (21) um den Spreizring (4) anzuordnen, drücken die seitlichen Anordnungen (42) des Spreizrings (4) auf die Verlängerungen des Fixiermittels (81), wodurch sich diese drehen und die Verdickung des Fixiermittels (81) nach oben und aus der Handschuhöffnung (21) kippt. Dadurch wird der Handschuh (2) nicht länger zwischen den Aufnahmevorrichtungen (8) gespannt und er kann am Spreizring (4) aus der Haltevorrichtung (5, 52) entnommen werden.
- Fig. 10: zeigt beispielhaft eine seitliche, schemenhafte Darstellung der Haltevorrichtung (5, 52) mit Einweghandschuhen (2). Dabei sind die Aufnahmevorrichtungen (8) der Haltevorrichtung (5, 52) an der Apparatur (3) angebracht. Die Handschuhe (2) sind typischerweise statisch an der Aufnahmevorrichtungen (8) und somit an der Haltevorrichtung (5, 52) angeordnet, wobei sie nach einer Handschuhentnahme ihre Position nicht verändern. Hingegen wird der Spreizring (4) (nicht dargestellt) - typischerweise mittels Computerprogramm - zu einem gespannten Handschuh (2) geführt, wo er auch aus der Haltevorrichtung (5, 52) entnommen werden kann.

## Patentansprüche

1. System (1) zum Einführen einer Hand in eine Handschuhöffnung (21) eines geöffneten Einweghandschuhs (2) und Anziehen des Einweghandschuhs (2) an die Hand, **dadurch gekennzeichnet, dass** das System (1)
a) eine Apparatur (3) mit führbarem Spreizring (4) zum Öffnen des Einweghandschuhs (2), und
b) eine Haltevorrichtung (5) zur Aufnahme und zum Spannen von mindestens einem Einweghandschuh (2)
umfasst, wobei die Haltevorrichtung (5) in der Apparatur (3) angeordnet ist oder reversibel angeordnet werden kann und mindestens einen Einweghandschuh (2) umfasst.

2. System (1) nach Anspruch 1, **dadurch gekennzeichnet, dass** die Apparatur (3) eine Bewegungsvorrichtung (6) umfasst, an welcher der Spreizring (4) angebracht ist, wobei die Bewegungsvorrichtung (6)
- den Spreizring (4) in einen in der Haltevorrichtung (5) gespannten Einweghandschuh (2) führen kann, wodurch der Handschuh (2) über einen Randbereich (41) des Spreizrings (4) angeordnet wird und sich der so durch den Spreizring (4) geöffnete Handschuh (2) von der Haltevorrichtung (5) löst und somit der Handschuh (2) durch den Spreizring (4) von der Haltevorrichtung (5) entnommen wird, und
- den Spreizring (4) mit dem daran angeordneten und gespannten Handschuh (2) in eine Position bringen kann, an welcher die Hand in den geöffneten Einweghandschuh (2) eingefügt werden kann,
wobei der Spreizring (4) in der Bewegungsvorrichtung (6) bevorzugt in einer S-Form geführt wird.

3. System (1) nach Anspruch 1 oder 2, **dadurch gekennzeichnet, dass** die Apparatur (3) eine verschliessbare Box (7) mit mindestens einer Öffnung (71) aufweist, die Haltevorrichtung (5) - gegebenenfalls reversibel - in der Box (7) angeordnet ist und der Spreizring (4), bevorzugt mittels Bewegungsvorrichtung (6), zur Öffnung (71) geführt werden kann, wobei die Apparatur (3) eine Vakuumpumpe (72) aufweist, mit welcher in der Box (7) bei geschlossener Öffnung (71) ein Unterdruck hergestellt werden kann.

4. System (1) nach Anspruch 3, **dadurch gekennzeichnet, dass** die Apparatur (3) mindestens eine Luftansaugglocke (73) umfasst, wobei die Luftansaugglocke (73) mit der Vakuumpumpe (72) verbunden ist und mindestens einen mit Luft gefüllten Handschuh (2) aufnehmen kann.

5. System (1) nach mindestens einem der Ansprüche 1 bis 4, **dadurch gekennzeichnet, dass** die Haltevorrichtung (5) zwei zueinander parallel angeordnete Aufnahmevorrichtungen (8) zur Aufnahme einer Vielzahl an Einweghandschuhen (2) aufweist und an jeder Aufnahmevorrichtung (8) eine Vielzahl an Fixiermitteln (81) angeordnet ist, wobei die Einweghandschuhe (2) mittels der Fixiermittel (81) zwischen die Aufnahmevorrichtungen (8) gespannt werden können.

6. System (1) nach Anspruch 5, **dadurch gekennzeichnet, dass**
i) der Spreizring (4) seitliche Anordnungen (42) umfasst, wobei, wenn der Handschuh (2) über den Randbereich (41) des Spreizrings (4) angeordnet wird, die seitlichen Anordnungen (42) auf die Fixiermittel (81), an denen der zu lösende Handschuh (2) fixiert ist, so einwirken können, dass sich der Handschuh (2) von den Fixiermitteln (81) und somit von der Haltevorrichtung (5) löst und der Handschuh (2) durch den Spreizring (4) von der Haltevorrichtung (5) entnommen werden kann, und/oder
ii) die Fixiermittel (81) an der Aufnahmevorrichtungen (8) so angebracht sind, dass die die seitlichen Anordnungen (42) auf die Fixiermittel (81) einwirken können, wobei die Fixiermittel (81) bevorzugt in Form
- einer Klemme vorliegen in welche der Handschuh (2) reversibel eingeklemmt sein kann, wobei die Aufnahmevorrichtungen (8) bevorzugt in Form von flächigen Aufnahmevorrichtungen vorliegen, oder
- von um eine Achse drehbare Fixiermittel (81) vorliegen, wobei die Aufnahmevorrichtungen (8) bevorzugt in Form von zylindrischen, stabförmigen Aufnahmevorrichtungen vorliegen, um welche sich die Fixiermittel (81) drehen können.

7. System (1) nach mindestens einem der Ansprüche 1 bis 6, **dadurch gekennzeichnet, dass** die Apparatur (3) einen Inkrement-Mechanismus (9) umfasst und die Haltevorrichtung (5) eine Vielzahl an Einweghandschuhen (2) aufnehmen und spannen kann, wobei der Inkrement-Mechanismus (9)
a) ein Inkrement-Mechanismus (91) darstellt, welcher die Haltevorrichtung (5), die Aufnahmevorrichtungen (8) und/oder die daran angeordneten Handschuhe (2) nach jeder Handschuhentnahme durch den Spreizring (4) um ein Inkrement bewegen kann, sodass der nachfolgende Handschuh (2) wieder am gleichen Ort angeordnet wird wie der zuvor entnommene Handschuh (2), und/oder
b) ein Inkrement-Mechanismus (92) darstellt, welcher den Spreizring und gegebenenfalls die Bewegungsvorrichtung (6) um ein Inkrement so steuert, dass nach einer Handschuhentnahme ein an der Haltevorrichtung (5) angeordneter nächster Handschuh (2) durch den Spreizring (4) von der Haltevorrichtung (5) entnommen werden kann,
wobei das Inkrement bevorzugt der Distanz von zwei benachbarten Handschuhen (2) entspricht.

8. System (1) nach mindestens einem der Ansprüche 1 bis 7, **dadurch gekennzeichnet, dass** die Haltevorrichtung (5)
i) eine Haltevorrichtung (51) mit Einweghandschuhen (2) darstellt, umfassend zwei zueinander parallel angeordnete Aufnahmevorrichtungen (8) mit Fixiermitteln (81), mindestens einer Einrastvorrichtung (82) sowie einem Behälter (83) zur Aufnahme einer Vielzahl von Einweghandschuhen (2), wobei
- die Aufnahmevorrichtung (8) und Fixiermittel (81) im Behälter (83) angeordnet sind und die Handschuhe (2) durch die Fixiermittel (81) zwischen den beiden Aufnahmevorrichtungen (8) gespannt sind,
- der Behälter (83) im Bereich der Handschuhöffnung (21) mindestens eines Handschuhs (2) offen ist,
- die Einrastvorrichtung (82) an mindestens einem Wandbereich des Behälters (83) angeordnet ist, und
- die Einrastvorrichtung (82) mit der Aufnahmevorrichtung (8) in Verbindung steht und mit dem Inkrement-Mechanismus (9) in Verbindung gebracht werden kann, wodurch bei Betätigung des Inkrement-Mechanismus (9) auch die Einrastvorrichtung (82) und somit die Aufnahmevorrichtung (8) betätigt wird,
oder
ii) eine Haltevorrichtung (52) mit Einweghandschuhen (2) darstellt, umfassend zwei zueinander parallel angeordnete Aufnahmevorrichtungen (8) mit Fixiermitteln (81), wobei die Handschuhe (2) durch die Fixiermittel (81) zwischen den beiden Aufnahmevorrichtungen (8) gespannt sind und mindestens ein Abstandshalter (84) die beiden Aufnahmevorrichtungen (8) so voneinander trennt, dass die Handschuhe (2) zwischen den Aufnahmevorrichtungen (8) gespannt sind.

9. System (1) nach mindestens einem der Ansprüche 1 bis 8, **dadurch gekennzeichnet, dass** das System (1) mindestens zwei nebeneinander angeordnete Apparaturen (3) und Haltevorrichtungen (5) aufweist, wodurch mindestens zwei Hände miteinander in je einen Einweghandschuh (2) eingeführt und angezogen werden können.

10. Verfahren zum Einführen einer Hand in einen geöffneten Einweghandschuh (2) und Anziehen des Einweghandschuhs (2) an die Hand mit dem System (1) nach mindestens einem der Ansprüche 1 bis 9, **dadurch gekennzeichnet, dass**
a) die Haltevorrichtung (5) mit mindestens einem gespannten Einweghandschuh (2) ausgerüstet wird, wobei die Haltevorrichtung (5) vor- oder nachher in der Apparatur (3) angeordnet wird,
b) der Spreizring (4) der Apparatur (3) in den mindestens einen in der Haltevorrichtung (5) gespannten Einweghandschuh (2) geführt wird, wodurch die Handschuhöffnung (21) geöffnet wird,
c) der geöffnete Handschuh (2) von der Haltevorrichtung (5) gelöst wird, und der Spreizring (4) mit dem geöffneten Handschuh (2) in eine Position gebracht wird, an welcher die Hand in den geöffneten Handschuh (2) eingefügt wird, und
d) der an der Hand angezogene Handschuh (2) vom Spreizring (4) gelöst wird und die Hand aus der Apparatur (3) entfernt.

11. Verfahren nach Anspruch 10, **dadurch gekennzeichnet, dass** der Spreizring (4) an der Bewegungsvorrichtung (6) angebracht wird, wobei die Bewegungsvorrichtung (6)
- den Spreizring (4) in die Handschuhöffnung (21) eines in der Haltevorrichtung (5) gespannten Einweghandschuh (2) führt, wodurch die Handschuhöffnung (21) über den Randbereich (41) des Spreizrings (4) angeordnet wird und sich der so durch den Spreizring (4) geöffnete Handschuh (2) von der Haltevorrichtung (5) löst und der Handschuh (2) durch den Spreizring (4) von der Haltevorrichtung (5) entnommen wird, und
- den Spreizring (4) mit dem daran angeordneten und gespannten Handschuh (2) in eine Position, insbesondere an die Öffnung (71), bringt, an welcher die Hand in den geöffneten Einweghandschuh (2) eingefügt werden kann,
wobei die Bewegungsvorrichtung (6) mit dem Spreizring (4) bevorzugt in einer S-Form geführt wird.

12. Verfahren nach Anspruch 10 oder 11, **dadurch gekennzeichnet, dass**
i) der Spreizring (4) seitliche Anordnungen (42) umfasst, wobei, wenn der Handschuh (2) über den Randbereich (41) des Spreizrings (4) angeordnet wird, die seitlichen Anordnungen (42) auf die Fixiermittel (81), an denen der zu lösende Handschuh (2) fixiert ist, so einwirken, dass sich der Handschuh (2) von den Fixiermitteln (81) und somit von der Haltevorrichtung (5) löst und der Handschuh (2) durch den Spreizring (4) von der Haltevorrichtung (5) entnommen wird, und/oder
ii) die Fixiermittel (81) an der Aufnahmevorrichtungen (8) so angebracht werden, dass die seitlichen Anordnungen (42) des Spreizrings (4) bei der Entnahme eines Handschuhs (2) auf die Fixiermittel (81) einwirken, dass sich der Handschuh (2) von den Fixiermitteln (81) löst, wobei die Fixiermittel (81) bevorzugt in Form
- einer Klemme vorliegen in welche der Handschuh (2) reversibel eingeklemmt sein kann, wobei die Aufnahmevorrichtungen (8) bevorzugt flächige Aufnahmevorrichtungen darstellen, oder
- von um eine Achse drehbare Fixiermittel (81) vorliegen, wobei die Aufnahmevorrichtungen (8) bevorzugt zylindrische, stabförmige Aufnahmevorrichtungen darstellen, um welche sich die Fixiermittel (81) drehen können.

13. Verfahren nach mindestens einem der Ansprüche 10 bis 12, **dadurch gekennzeichnet, dass**
- die Bewegungsvorrichtung (6) den Spreizring (4) mit dem am Spreizring (4) angeordneten und geöffneten Handschuh (2) an die Öffnung (71) der verschliessbaren Box (7) heranführt, sodass die vom Handschuh (2) abgewandte Seite des Spreizrings (4) die Öffnung (71) umschliesst, wodurch der Handschuh (2) die Abgrenzung zwischen Innenbereich und Aussenbereich der Box (7) darstellt,
- mittels Vakuumpumpe (72), gegebenenfalls über die Luftansaugglocke (73), in der Box (7) ein Unterdruck erzeugt wird, wodurch der Handschuh (2) nach innen, gegebenenfalls in die Luftansaugglocke (73), gesogen wird, wodurch sich der Handschuh (2) vollständig öffnet,
- von der Aussenseite der Box (7) die Hand durch die Öffnung (71) in den geöffneten Handschuh (2) eingefügt wird, und
- die Vakuumpumpe (72) gestoppt, der Handschuh (2) vom Spreizring (4) gelöst und die Hand mit dem angezogenen Handschuh (2) aus der Box (7) entfernt wird.

14. Verfahren nach mindestens einem der Ansprüche 10 bis 12, **dadurch gekennzeichnet, dass** die Apparatur (3)
i) einen Inkrement-Mechanismus (91) und die Haltevorrichtung (5) eine Haltevorrichtung (51) mit einer Einrastvorrichtung (82) darstellt, wobei die Haltevorrichtung (51) so in die Apparatur (3) eingebracht wird, dass die Einrastvorrichtung (82) mit dem Inkrement-Mechanismus (91) verbunden wird, wodurch sich durch Betätigung des Inkrement-Mechanismus (91) die Position der Handschuhe (2) in der Haltevorrichtung (51) verändert, oder
ii) einen Inkrement-Mechanismus (92) umfasst und die Haltevorrichtung (5) eine Haltevorrichtung (52) darstellt, wobei der Inkrement-Mechanismus (92), gegebenenfalls über die Bewegungsvorrichtung (6), mit dem Spreizring (4) verbunden wird, wodurch sich die Position des Spreizrings (4) bei jeder Entnahme eines Handschuhs (2) verändert.

15. Verwendung des Systems (1) nach mindestens einem der Ansprüche 1 bis 9 zum Einführen mindestens einer Hand in einen geöffneten Einweghandschuh (2) und Anziehen des mindestens einen Einweghandschuhs (2) an eine Hand, insbesondere im Gesundheitswesen, in Spitälern, bei Arzt- und Zahnarztpraxen, in Laboratorien wie chemische, biotechnologische und/oder biologische Laboratorien, bei der Produktion, Verarbeitung und/oder beim Verkauf von Lebensmittels, in Grossküchen, Frisiersalons, insbesondere beim Färben von Haaren, in Tattoo-Studios sowie für Reinigungsarbeiten jeglicher Art.
